# FASCICULE DE BREVET EUROPEEN

(11) **EP 4 127 636 B1**
(45) Date de publication et mention de la délivrance du brevet: **11.06.2025**
(21) Numéro de dépôt: 21723320.4
(22) Date de dépôt: 01.04.2021
(51) Int. Cl.: G01L 1/26, A61F 2/00, G01L 5/00

(54) **SYSTEME DE PROTECTION D'UN CAPTEUR POUR UNE MESURE DE FORCE ET DISPOSITIF OCCLUSIF IMPLANTABLE COMPRENANT UN TEL SYSTEME DE PROTECTION DU CAPTEUR**
SYSTEM ZUM SCHUTZ EINES SENSORS FÜR EINE KRAFTMESSUNG UND IMPLANTIERBARE OKKLUSIONSVORRICHTUNG MIT EINEM SOLCHEN SYSTEM ZUM SCHUTZ DES SENSORS
SYSTEM FOR PROTECTING A SENSOR FOR A FORCE MEASUREMENT AND IMPLANTABLE OCCLUSIVE DEVICE COMPRISING SUCH A SYSTEM FOR PROTECTING THE SENSOR

(30) Priorité: 01.04.2020 FR 2003237
(43) Date de publication de la demande: 08.02.2023
(73) Titulaire: Uromems, 38320 Eybens (FR)
(72) Inventeur: LAMRAOUI, Hamid, 38000 GRENOBLE (FR); MIR, Riaz, 38600 FONTAINE (FR); MARIEN, Marc, 38700 LA TRONCHE (FR); LE LOC'H, Clémentine, 38240 MEYLAN (FR)
(74) Mandataire: Regimbeau
(86) Numéro de dépôt international: PCT/FR2021/050578
(87) Numéro de publication internationale: WO 2021/198622

(56) Documents cités:
- WO-A1-2016/083428
- US-A- 5 287 757

## Description

### DOMAINE DE L'INVENTION

L'invention concerne un système de protection d'un capteur pour une mesure de force, ainsi qu'un dispositif hydraulique implantable comprenant un tel système de protection du capteur.

### ETAT DE LA TECHNIQUE

Il existe des dispositifs hydrauliques médicaux destinés à être implantés dans un corps humain ou animal pour lutter contre les dysfonctionnements érectiles ou procurant une occlusion sélective d'un conduit anatomique, tel qu'un urètre, un col vésical, un côlon, un rectum ou un estomac par exemple.

Les dispositifs occlusifs comprennent généralement une manchette entourant ledit conduit anatomique et adaptée pour exercer une compression sur ledit conduit.

La compression exercée par la manchette est commandée par une unité de commande électromécanique.

Pour permettre une régulation de la pression exercée sur le conduit à occlure, la manchette gonflable est en liaison fluidique avec un réservoir de fluide couplé à un actionneur configuré pour injecter du fluide du réservoir vers la manchette (afin d'augmenter la pression exercée sur le conduit anatomique) ou de la manchette vers le réservoir (afin de réduire la pression exercée sur le conduit anatomique). L'ensemble de la manchette gonflable, du réservoir et de la liaison fluidique entre eux forme un circuit fluidique.

Dans un tel système occlusif, il peut être nécessaire de mesurer la pression dans la manchette gonflable ou en un autre point du circuit fluidique, par exemple pour vérifier la pression lorsque l'actionneur est désactivé, ou encore pour contrôler la pression exercée par ledit actionneur.

Le document WO 2016/083428 décrit un dispositif occlusif implantable qui comprend un circuit fluidique incluant une manchette occlusive gonflable, un réservoir de fluide à volume variable comprenant une partie fixe et une partie mobile, en liaison fluidique avec la manchette, et un actionneur couplé mécaniquement à la partie mobile du réservoir de sorte à déplacer linéairement ladite partie mobile par rapport à la partie fixe pour ajuster le volume du réservoir et induire ainsi un transfert de fluide entre le réservoir et la manchette.

L'actionneur et le réservoir à volume variable sont agencés dans un boîtier étanche contenant un gaz.

Un capteur de force agencé dans le boîtier est lié mécaniquement à la partie mobile du réservoir pour mesurer une force de traction et/ou de compression dans la direction de déplacement de la partie mobile du réservoir.

La force mesurée par ce capteur permet de calculer la pression dans le circuit fluidique.

Cependant, des charges excessives appliquées au capteur, notamment en cas d'élévation de pression dans le réservoir, pourraient l'endommager.

### EXPOSE DE L'INVENTION

Un but de l'invention est donc de concevoir un système de protection d'un capteur pour la mesure de force dans un dispositif occlusif implantable permettant de protéger le capteur de force contre des charges excessives.

A cet effet, un premier objet de l'invention concerne un système de protection d'un capteur pour une mesure de force comprenant :
- un capteur de mesure de force, adapté pour mesurer au moins une force de traction ou une force de compression exercée selon un axe longitudinal du capteur, ledit capteur étant adapté pour être solidarisé à une partie mobile d'un réservoir de fluide, et
- un élément élastique précontraint agencé pour solliciter le capteur de force dans un sens opposé à ladite force exercée, ledit élément élastique étant déformable dans le sens de la force exercée de sorte à protéger le capteur d'au moins une force de compression ou de traction supérieure à un seuil.

De manière particulièrement avantageuse, le système comprend en outre une butée, le capteur de mesure de force étant mobile selon ledit axe jusqu'à ladite butée dans le sens de la force exercée à l'encontre de la sollicitation de l'élément élastique.

De préférence, l'élément élastique est précontraint à une valeur de force déterminée, dite force de précontrainte, ladite force de précontrainte étant inférieure à une force maximale pouvant être supportée par le capteur de mesure de force, de sorte que le capteur de mesure de force est adapté pour ne se déplacer vers la butée qu'au-delà de ladite force de précontrainte

Dans certains modes de réalisation, le système comprend en outre une roue dentée solidaire du capteur de mesure de force.

Le système peut en outre comprendre un roulement à billes solidaire du capteur de mesure de force.

Pour protéger le capteur contre une force de compression excessive, la butée est agencée d'un côté du capteur de mesure de force opposé au réservoir de fluide. Par ailleurs, l'élément élastique est agencé d'un côté du capteur de mesure de force opposé au réservoir de fluide.

Inversement, pour protéger le capteur contre une force de traction excessive, la butée est agencée du même côté du capteur de force que le réservoir de fluide. Par ailleurs, l'élément élastique est agencé du même côté du capteur de mesure de force que le réservoir de fluide.

De manière particulièrement avantageuse, l'élément élastique est solidaire du capteur de mesure de force de manière à permettre une mesure d'une force de traction et/ou de compression par le capteur de mesure de force.

Dans certains modes de réalisation, l'élément élastique précontraint est une rondelle ressort.

Dans une forme d'exécution préférée, ladite rondelle ressort est une rondelle élastique ondulée.

Dans certains modes de réalisation, le capteur de mesure de force comprend une portion annulaire présentant une réduction d'épaisseur, ladite portion étant apte à fléchir sous l'application d'une force axiale de compression ou de traction.

De manière avantageuse, le capteur de mesure de force est en appui sur l'élément élastique précontraint par une portion périphérique extérieure à la portion présentant la réduction d'épaisseur.

Dans certains modes de réalisation, le capteur comprend une jauge de contrainte collée sur ladite portion annulaire présentant la réduction d'épaisseur.

Un autre objet concerne un dispositif médical adapté pour être implanté dans un corps humain ou animal, comprenant :
(a) un circuit fluidique comprenant :
   - une manchette occlusive gonflable contenant un volume variable d'un fluide, adaptée pour entourer au moins une partie d'un conduit naturel à occlure,
   - un réservoir à volume variable rempli d'un fluide, ledit réservoir comprenant une partie fixe et une partie mobile,
   - une liaison fluidique entre le réservoir et la manchette occlusive,
(b) un actionneur couplé mécaniquement à la partie mobile du réservoir de sorte à déplacer linéairement ladite partie mobile par rapport à la partie fixe pour ajuster le volume du réservoir,
   l'actionneur et le réservoir à volume variable étant agencés dans un boîtier étanche,
(c) un système de protection d'un capteur pour une mesure de force tel que décrit ci-dessus, le capteur de mesure de force étant solidaire de la partie mobile du réservoir à volume variable.

Dans certains modes de réalisation, la partie mobile du réservoir à volume variable est un soufflet.

De manière particulièrement avantageuse, ledit soufflet comprend une paroi solidaire d'une vis d'entraînement, ladite vis d'entraînement étant couplée par une liaison hélicoïdale à une roue dentée apte à être entraînée en rotation par l'actionneur, le capteur de mesure de force étant agencé autour de la roue dentée par l'intermédiaire d'un roulement à billes.

Dans certains modes de réalisation, la roue dentée et le capteur sont agencés dans une boîte à engrenages, le capteur étant maintenu contre l'élément élastique par une bague de fixation.

Un autre objet concerne une méthode de protection d'un capteur de mesure de force adapté pour mesurer au moins une force de traction ou une force de compression exercée selon un axe longitudinal du capteur et pour être solidarisé à une partie mobile d'un réservoir de fluide, ladite méthode comprenant au moins les étapes consistant à :
- fournir un système de protection dudit capteur tel que décrit ci-dessus,
- exercer une force de traction ou de compression selon l'axe du capteur de sorte que (i) tant que ladite force est inférieure à la force de précontrainte de l'élément élastique, le capteur se déforme pour mesurer la force exercée et (ii) lorsque ladite force devient supérieure à ladite force de précontrainte, le capteur se déplace jusqu'à une butée.

De manière particulièrement avantageuse, le capteur atteint la butée lorsque la force exercée est inférieure à une force de détérioration du capteur.

Par ailleurs, le capteur ne se déplace vers la butée qu'au-delà de la force de précontrainte.

### BREVE DESCRIPTION DES FIGURES

D'autres caractéristiques et avantages de l'invention ressortiront de la description détaillée qui va suivre, en référence aux dessins annexés, sur lesquels :
- la figure 1 est un schéma de principe d'un dispositif occlusif implantable ;
- la figure 2A est un schéma de principe du système de sécurité du capteur de force à l'état libre ;
- la figure2B est un schéma de principe du système de sécurité du capteur de force de la figure 2A soumis à une force de compression ;
- la figure 3A est un schéma de principe du système de sécurité du capteur de force de la figure 2B lorsque la force de compression est inférieure à un seuil ;
- la figure 3B est un schéma de principe du système de sécurité du capteur de force de la figure 2B lorsque la force de compression est supérieure audit seuil ;
- la figure 4 est un schéma de principe d'un second mode de réalisation du système de sécurité du capteur de force à l'état libre.

Pour des raisons de lisibilité des figures, les dessins n'ont pas nécessairement été réalisés à l'échelle.

### DESCRIPTION DETAILLEE DE MODES DE REALISATION

Le dispositif occlusif implantable comprend une manchette occlusive gonflable contenant un volume variable d'un fluide, destinée à entourer au moins une partie d'un conduit naturel à occlure, et un réservoir à volume variable rempli d'un fluide.

Ledit réservoir comprend une partie fixe et une partie mobile, le déplacement de la partie mobile faisant varier le volume du réservoir.

A cet effet, le dispositif occlusif comprend un actionneur couplé mécaniquement à la partie mobile du réservoir de sorte à déplacer linéairement ladite partie mobile par rapport à la partie fixe pour ajuster le volume du réservoir. L'actionneur peut notamment comprendre un moteur électromagnétique et un réducteur. L'actionneur est commandé par un dispositif de contrôle de la pression de la manchette mettant en oeuvre un capteur de force dont l'agencement sera décrit plus bas.

Pour chaque valeur de volume du réservoir, la partie mobile présente une surface de pression effective connue, qui peut être constante ou variable suivant les modes de réalisation.

Le dispositif occlusif comprend en outre une liaison fluidique (typiquement une tubulure) entre le réservoir et la manchette occlusive.

Ainsi, une variation de volume du réservoir entraîne un ajout ou un retrait de fluide dans la manchette, faisant ainsi augmenter ou diminuer la compression exercée sur le conduit entouré par la manchette.

L'ensemble formé du réservoir à volume variable, de la manchette occlusive et de la liaison fluidique est appelé circuit fluidique dans la suite de la description.

Le dispositif comporte en outre une source d'énergie rechargeable ou non permettant d'alimenter les différents composants. Dans une configuration particulière la source d'énergie est à l'extérieur du corps humain et transmet l'énergie sans fil au dispositif implanté, par exemple par couplage inductif.

Le réservoir à volume variable, l'actionneur et, le cas échéant, la source d'énergie, sont agencés dans un boîtier destiné à être implanté dans le corps du patient. Le boîtier contient un gaz, par exemple de l'air. Ledit boîtier doit être étanche pour éviter tout transfert de fluide ou de gaz de ou vers le milieu intracorporel. Le boîtier est en un matériau biocompatible et peut par exemple être réalisé en titane implantable et scellé par soudure laser. Un contrôle de l'étanchéité peut notamment être réalisé à l'hélium pour s'assurer de l'étanchéité totale du boîtier pendant la période pour laquelle le dispositif est implanté.

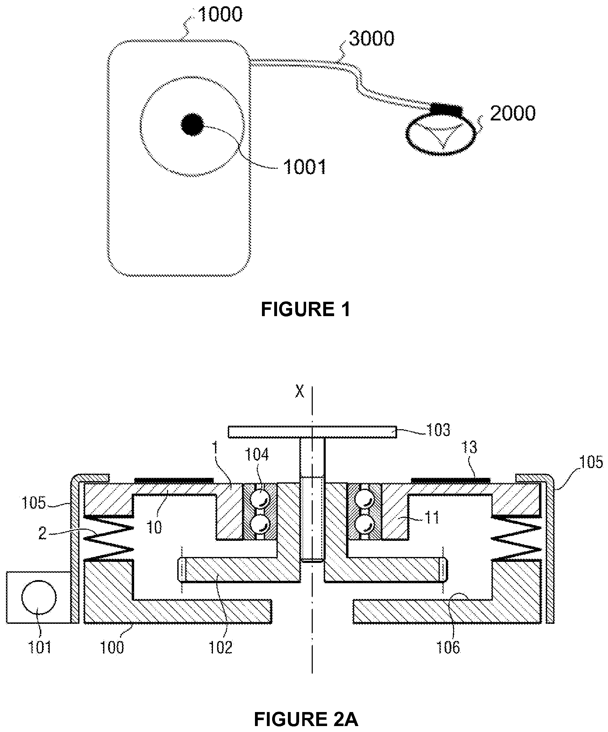

La figure 1 illustre un dispositif occlusif implantable comprenant un tel boîtier 1000, une manchette occlusive 2000 et une tubulure 3000 reliant le réservoir à volume variable situé à l'intérieur du boîtier 1000 et la manchette 2000.

Selon un mode de réalisation, le boîtier peut comprendre un port de ponction perforable 1001 agencé dans une paroi du réservoir à volume variable. Un utilisateur peut percer ledit port au moyen de l'aiguille d'une seringue afin d'ajouter ou éventuellement de retirer du fluide dans le réservoir.

Selon un mode de réalisation préféré, le réservoir à volume variable comprend un soufflet assemblé dans le boîtier, le soufflet et le boîtier étant par exemple réalisés en titane implantable. Le réservoir à volume variable est alors constitué du soufflet (faisant office de partie mobile), d'une paroi du boîtier et d'un capot faisant office, avec ladite paroi du boîtier, de partie fixe. Le réservoir comprend en outre un orifice permettant de transférer le fluide de et vers l'extérieur du réservoir.

Le soufflet présente l'avantage d'assurer une étanchéité totale de l'implant tout en autorisant le mouvement de la paroi mobile. Cependant, la présente invention n'est pas limitée à l'utilisation d'un soufflet pour former le réservoir à volume variable. Ainsi, l'homme du métier pourra mettre en oeuvre pour réaliser le réservoir à volume variable un piston ou une membrane roulante ou déformable.

Dans le cas d'un soufflet, la surface de pression effective est considérée constante et est donnée par le fabricant. Pour une membrane roulante, la surface de pression effective varie en fonction la position de la membrane roulante et est donnée par le fabricant pour différentes valeurs de course. Dans le cas d'un piston coulissant sans frottement dans un cylindre, la surface de pression effective est égale à la surface frontale du piston.

L'actionneur peut être choisi parmi tout système électromécanique permettant de transformer une énergie électrique en un mouvement mécanique avec la puissance requise pour permettre le déplacement à une force et à une vitesse requise de la partie mobile du réservoir à volume variable. On peut citer pour exemple, parmi les actionneurs connus de l'homme du métier, les actionneurs piézo-électriques, les moteurs électromagnétiques avec ou sans balais (dans le cas d'un moteur sans balais, celui-ci peut être constitué de deux pôles ou de quatre pôles) couplés ou non à un réducteur, les polymères électro-actifs ou bien les alliages à mémoire de forme.

Le boîtier renferme également un capteur en liaison mécanique avec la paroi mobile du réservoir à volume variable de sorte à mesurer une force de compression et/ou de traction dans la direction de déplacement de la partie mobile du réservoir. Ce capteur est appelé dans la suite du texte « capteur de mesure de force » ou « capteur de force ». Sauf indication contraire, ce capteur peut être simplement désigné par le terme « capteur » dans un souci de concision.

Le capteur de force est adapté pour se défléchir sous l'application d'une force de compression ou de traction. Cette déformation peut être mesurée par exemple au moyen d'une jauge de déformation ou jauge de contrainte, et la force appliquée est déterminée à partir de la déformation ainsi mesurée.

De manière particulièrement avantageuse, le capteur est monté sur un élément élastique qui le sollicite dans un sens opposé à celui de la force de compression. Cet élément élastique procure deux avantages. D'une part, il permet de protéger mécaniquement la déflexion du capteur de force dans des cas d'application d'une force de compression excessive, où la mesure de force n'est pas nécessairement souhaitée. D'autre part, il permet d'optimiser la sensibilité de la lecture de la force mesurée par le capteur dans la plage de force souhaitée dans le cadre d'un usage normal du dispositif.

Lorsque l'on souhaite protéger le capteur contre une force de traction excessive, l'élément élastique est agencé pour solliciter le capteur dans un sens opposé à celui de la force de traction.

Eventuellement, on peut protéger le capteur à la fois contre des forces de traction et de compression excessives en disposant deux éléments élastiques de part et d'autre du capteur de force.

L'élément élastique est précontraint à une valeur de force déterminée, appelée « force de précontrainte » dans la suite du texte. Cette valeur est choisie pour correspondre à une force qui est inférieure à la force maximale que peut supporter le capteur avant de se détériorer, de sorte que le capteur ne se déplace qu'au-delà de cette force de précontrainte pour arriver en butée et ainsi être protégé contre la détérioration.

La position de la butée est déterminée en fonction des paramètres de raideur de l'élément élastique, de la force de précontrainte souhaitée, de la déformation maximale du capteur et de sorte à atteindre une force inférieure à la force maximale nécessaire pour la protection du capteur dans cette position.

Le fait de précontraindre l'élément élastique est particulièrement avantageux lors la déformation du capteur pour éviter la mise en contact prématurée avec la butée alors que l'on est dans une phase de mesure de la force. En effet, lors de la déformation du capteur, ce dernier se trouve placé très proche de la butée avec un contact possible, ce qui pourrait, en l'absence de l'élément élastique, à la fois impacter la mesure de force et/ou nuire au déplacement de la partie mobile du réservoir à volume variable comme expliqué ci-après.

Avantageusement, un tel système permet donc une mesure de force fiable dans une plage de force suffisante sans détérioration du capteur.

Tant que la force exercée sur le capteur est inférieure à cette valeur, seul le capteur se déforme, ce qui permet une mesure directe de la force.

Si la force exercée sur le capteur excède ladite valeur, l'élément élastique se comprime et le capteur déformé se déplace jusqu'à atteindre la butée. La mesure de la force reste possible, mais est moins précise.

Une fois la butée atteinte, le capteur ne se déforme plus et la mesure de force n'est donc plus possible. Toutefois, dans cette position, le capteur de force est protégé puisque la constante de raideur du ressort, la force de précontrainte du ressort, la déformation du capteur et la distance parcourue par le capteur au-delà de la valeur de force déterminée sont choisies et calculées pour que la force subie par le capteur soit inférieure à la force maximale qu'il peut supporter avant de se détériorer.

En d'autres termes, on peut distinguer trois phases de fonctionnement du capteur, dans le sens d'une force de compression croissante.

Dans une première phase, la force exercée sur le capteur (et mesurée par celui-ci) est comprise entre zéro et la force de précontrainte, et la déflexion du capteur est comprise entre zéro et une première valeur notée X1. Cette première phase correspond à une situation d'usage normal du dispositif où l'on souhaite mesurer la force. La force mesurée par le capteur est donc fiable.

Dans une deuxième phase, la force exercée sur le capteur est comprise entre la force de précontrainte et une force limite, qui est inférieure à la force maximale que le capteur peut subir avant détérioration, et la déflexion du capteur est comprise entre X1 et une seconde valeur notée X2 (supérieure à X1). Dans cette deuxième phase, la valeur de force mesurée par le capteur est moins fiable que dans la première phase. Du fait de cette imprécision, on ne cherche pas à mesurer la force exercée.

Dans une troisième phase, la force exercée sur le capteur est supérieure ou égale à la force limite. Le capteur étant arrivé en butée, sa déflexion reste égale à X2.

Dans une forme d'exécution particulièrement avantageuse, l'élément élastique se présente sous la forme d'une rondelle ressort.

Dans certains modes de réalisation, la rondelle ressort est une rondelle ondulée. Une telle rondelle est découpée dans un feuillard métallique plat, par exemple d'acier ou de cuivre, puis formée pour présenter des ondulations. A l'état libre, non contraint, la rondelle présente une hauteur égale à l'amplitude des ondulations. La rondelle ondulée est déformable élastiquement jusqu'à adopter une forme quasi plane. La rondelle ondulée est préférée parmi les autres types de rondelles ressorts pour sa compacité et sa faible raideur.

Dans d'autres modes de réalisation, la rondelle ressort est une rondelle de type Belleville. Une telle rondelle est découpée dans un feuillard métallique plat, par exemple d'acier ou de cuivre, puis formée pour présenter une forme de cône tronqué. A l'état libre, non contraint, la rondelle présente une hauteur égale à la distance entre la base et le sommet du cône tronqué. La rondelle Belleville est déformable élastiquement jusqu'à adopter une forme quasi plane.

Par rapport à un ressort hélicoïdal ou une combinaison de plusieurs ressorts hélicoïdaux, un avantage de la rondelle ressort est qu'elle présente un encombrement réduit tout en ayant une raideur faible répondant aux exigences de forces souhaitées pour la mesure selon l'application médicale. Par ailleurs, elle exerce une force uniforme sur le capteur, ce qui garantit une bonne précision de la mesure de force en évitant d'avoir des efforts parasites dans la mesure, ce qui pourrait par exemple être le cas si on mettait en oeuvre une solution avec plusieurs ressorts hélicoïdaux répartis à différents emplacements de la circonférence au lieu de la rondelle ressort qui couvre toute la périphérie.

Une telle rondelle est donc particulièrement bien adaptée aux contraintes de miniaturisation d'un dispositif implantable.

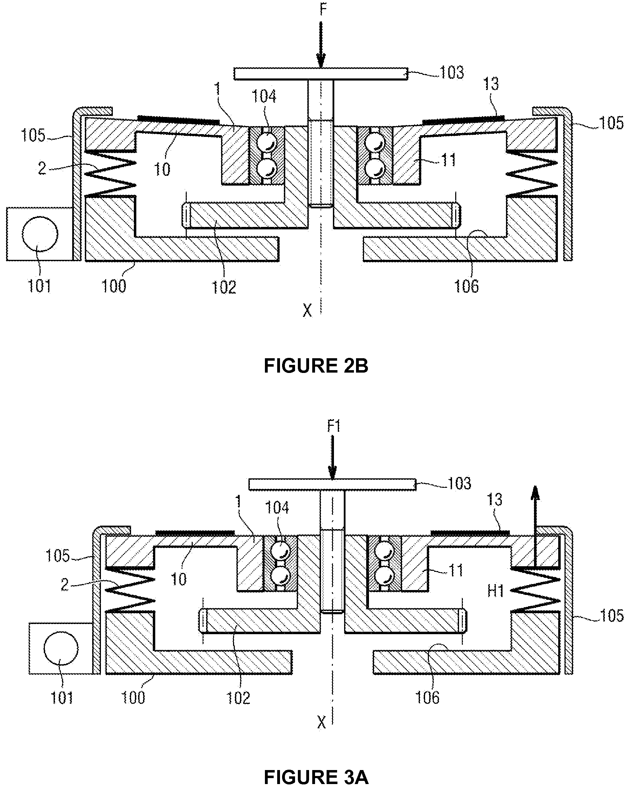

Les figures 2A et 2B illustrent un schéma de principe d'une partie de l'intérieur du boîtier au niveau de l'actionneur avec le capteur de force respectivement à l'état libre, c'est-à-dire non soumis à une force de compression et soumis à une force de compression.

Le réservoir à volume variable comprend une partie mobile qui, dans ce mode de réalisation, est un soufflet (non illustré).

Le soufflet présente une paroi solidaire d'une vis d'entraînement 103. Par exemple, l'extrémité de la vis 103 comprend une collerette plane qui peut être collée sur la paroi du soufflet.

Le boîtier contient en outre un actionneur 101 comprenant un moteur couplé à un réducteur.

Le réducteur est couplé à une roue dentée 102 qui est elle-même couplée à la vis d'entraînement 103 par une liaison hélicoïdale. La rotation de la roue 102 entraîne alors la vis d'entraînement en translation selon l'axe X, ce qui a pour effet de déplacer la paroi du soufflet en translation selon l'axe X, le sens de déplacement dépendant du sens de rotation du moteur.

Le capteur de force 1 est agencé autour de la roue dentée 102 par l'intermédiaire d'un roulement à billes 104. Le capteur est ainsi libre vis-à-vis du mouvement de rotation.

Le capteur présente une forme annulaire, dont une portion 10 présente une réduction d'épaisseur qui la rend apte à fléchir sous l'application d'une force exercée selon l'axe X. Le capteur comprend une jauge de contrainte 13 annulaire en contact avec la portion 10.

Le capteur comprend un fût central 11 présentant un orifice traversant pour son assemblage sur le roulement à billes 104.

La roue dentée 102 et le capteur de force 1 sont logés dans une boîte à engrenages 100.

Dans ce logement, le capteur de force 1 est monté entre une rondelle ressort 2 et une bague de fixation 105. La bague de fixation 105 ferme la boîte à engrenages 100 tout en permettant le passage de la vis d'entraînement 103 et maintient le capteur de force en contact avec la rondelle ondulée 2 tout en assurant la précontrainte souhaitée de la rondelle ondulée. Dans le mode de réalisation illustré, la rondelle ressort est une rondelle ondulée mais, de manière alternative (non illustrée), la rondelle ressort pourrait être une rondelle Belleville.

La rondelle ondulée précontrainte 2 exerce sur le capteur de force une force selon l'axe X de sens opposé à celui d'une force de compression. Cette force peut être exprimée comme le produit de la raideur de la rondelle par la différence de hauteur résultant d'une compression de la rondelle.

La liaison entre le capteur de force 1 et la rondelle ondulée 2 est effectuée au niveau d'une portion périphérique du capteur de force, extérieure à la portion 10 qui présente la réduction d'épaisseur. Cette portion périphérique est fixe tant que la force appliquée est inférieure à la force de précontrainte de la rondelle ressort, tandis que la portion centrale du capteur de force incluant un filetage (non illustré) est apte à se déplacer sous l'effet d'une force F exercée selon l'axe X, par fléchissement de la portion 10 (cf. figure 2B).

Lorsque la force appliquée est supérieure à la force de précontrainte, cette portion périphérique du capteur de force se déplace vers une butée détaillée ci-après en comprimant la rondelle ressort.

A cet effet, un espace est donc ménagé dans la direction de l'axe X entre le fond 106 de la boîte à engrenages 100 et la surface inférieure de l'ensemble capteur de force 1 - roulement à billes 104 - roue dentée 102, cet espace définissant une course maximale de l'ensemble capteur de force 1 - roulement à billes 104 - roue dentée 102 sous l'effet d'une force de compression. Le fond 106 de la boîte à engrenages forme la butée susmentionnée.

Lorsque cette butée est atteinte, la déflexion du capteur est impossible. Par ailleurs, du fait du frottement de la roue dentée 102 contre le fond 106, le mouvement de la roue dentée et du soufflet est bloqué.

La rondelle ondulée, combinée à la butée, remplit donc une fonction de sécurité, destinée à protéger le capteur contre une force de compression élevée susceptible de conduire à une déflexion excessive du capteur de force.

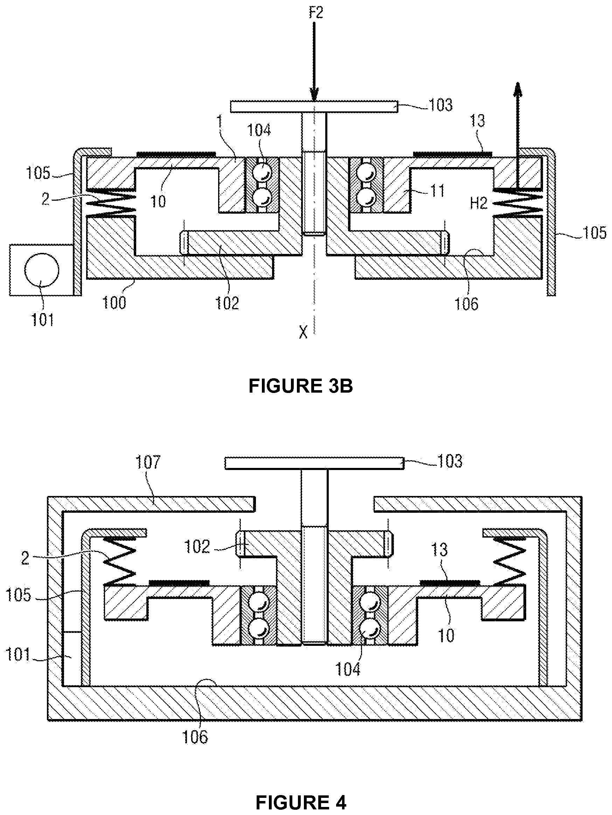

En effet, comme illustré sur la figure 3A, tant que la force de compression (notée F1) est inférieure à la valeur de la force de précontrainte de la rondelle ondulée, le capteur fléchit et la déflexion engendrée est mesurée par la jauge de contrainte 103 pour être convertie en une valeur de force convertible en valeur de pression ; la hauteur de la rondelle ondulée reste égale à la hauteur initiale H1 (vue de gauche). En revanche, si la force de compression (notée F2) excède ladite valeur de la précontrainte, l'ensemble capteur de force - roulement à billes - roue dentée se déplace selon l'axe X dans le sens de la force de compression, la rondelle ondulée se comprime et sa hauteur devient H2 inférieure à H1 (cf. figure 3B). La mesure de la force reste possible, mais moins précise. Lorsque la force atteint une certaine limite, l'ensemble capteur - roulement à billes - roue dentée arrive en butée contre le fond 106 de la boîte à engrenages 100 ce qui permet de limiter le déplacement de l'ensemble capteur de force - roulement à billes - roue dentée selon l'axe X dans le sens de la force de compression et donc de protéger le capteur de force (ainsi que le roulement à billes et la butée) contre des compressions trop élevées qui pourraient l'endommager.

Ce système de protection est actif uniquement contre les forces de compression excessives, correspondant typiquement à une surpression de fluide dans le réservoir à volume variable.

Cependant, comme expliqué plus haut, on peut concevoir sur le même principe un système de protection du capteur contre des forces de traction excessives.

Un tel système est représenté schématiquement sur la figure 4.

Les signes de référence identiques à ceux des figures précédentes représentent des composants identiques ou remplissant la même fonction. On ne décrira donc ici que les aspects spécifiques au mode de réalisation de la figure 4. L'élément élastique 2 est agencé entre la bague de fixation 105 et le capteur de force 1, afin de solliciter le capteur de force vers le fond 106 de la boîte à engrenages, c'est-à-dire dans le sens opposé à une force de traction. La jauge de contrainte 13 reste positionnée au niveau de la portion 10 présentant une restriction d'épaisseur. Dans ce cas, la butée peut être formée par une paroi supérieure 107 de la boîte à engrenages opposée au fond 106, et est atteinte lorsque la force de traction est inférieure à la force de détérioration du capteur.

Eventuellement, il serait possible de combiner les modes de réalisation des figures 2-3 et 4 pour protéger le capteur de force à la fois contre des forces de traction et des forces de compression excessives, en mettant en oeuvre deux éléments élastiques et deux butées.

L'homme du métier est à même de dimensionner la rondelle ondulée en fonction de la force acceptable par le capteur de force.

Dans le document WO 2016/083428, il est fait mention d'un système de précontrainte comprenant un ressort, mais ce système remplit une fonction différente de celle recherchée dans la présente invention. Ce système de précontrainte peut être mis en oeuvre lorsque le capteur ne permet de mesurer que des forces de compression, afin de créer un décalage ou « offset » sur le capteur de force permettant ainsi de mesurer également des forces de traction. De fait, dans le document WO 2016/083428, ce ressort n'est pas agencé du même côté du capteur que la rondelle ondulée selon la présente invention, et exerce une force de sens opposé à la force exercée par la rondelle ondulée. Par ailleurs, lorsque ce système de précontrainte est mis en oeuvre, le capteur n'est pas solidaire de la partie mobile du réservoir de fluide, contrairement à la présente invention.

### REFERENCES

WO 2016/083428

## Revendications

1. Système de protection d'un capteur pour une mesure de force comprenant :
- un capteur de mesure de force (1), adapté pour mesurer au moins une force de traction ou une force de compression exercée selon un axe longitudinal (X) du capteur, ledit capteur étant adapté pour être solidarisé à une partie mobile d'un réservoir de fluide, et
- un élément élastique précontraint (2) agencé pour solliciter le capteur de force (1) dans un sens opposé à ladite force exercée, ledit élément élastique étant déformable dans le sens de la force exercée de sorte à protéger le capteur d'au moins une force de compression ou de traction supérieure à un seuil.

2. Système selon la revendication 1, comprenant en outre une butée (106, 107), le capteur de mesure de force (1) étant mobile selon ledit axe (X) jusqu'à ladite butée dans le sens de la force exercée à l'encontre de la sollicitation de l'élément élastique (2).

3. Système selon la revendication 2, dans lequel l'élément élastique (2) est précontraint à une valeur de force déterminée, dite force de précontrainte, ladite force de précontrainte étant inférieure à une force maximale pouvant être supportée par le capteur de mesure de force, de sorte que le capteur de mesure de force est adapté pour ne se déplacer vers la butée qu'au-delà de ladite force de précontrainte.

4. Système selon l'une des revendications 1 à 3, comprenant en outre une roue dentée (102) solidaire du capteur de mesure de force (1).

5. Système selon l'une des revendications 1 à 4, comprenant en outre un roulement à billes (104) solidaire du capteur de mesure de force (1).

6. Système selon l'une des revendications 2 à 5, dans lequel la butée (106) est agencée d'un côté du capteur de mesure de force (1) opposé au réservoir de fluide.

7. Système selon l'une des revendications 1 à 6, dans lequel l'élément élastique (2) est agencé d'un côté du capteur de mesure de force (1) opposé au réservoir de fluide.

8. Système selon l'une des revendications 1 à 7, dans lequel l'élément élastique (2) est solidaire du capteur de mesure de force de manière à permettre une mesure d'une force de traction et/ou de compression par le capteur de mesure de force.

9. Système selon l'une des revendications 1 à 8, dans lequel l'élément élastique précontraint (2) est une rondelle ressort.

10. Système selon la revendication 9, dans lequel la rondelle ressort est une rondelle élastique ondulée.

11. Système selon l'une des revendications 1 à 10, dans lequel le capteur de mesure de force (1) comprend une portion (10) annulaire présentant une réduction d'épaisseur, ladite portion (10) étant apte à fléchir sous l'application d'une force axiale.

12. Système selon la revendication 11, dans lequel le capteur de mesure de force (1) est en appui sur l'élément élastique (2) précontraint par une portion périphérique extérieure à la portion (10) présentant la réduction d'épaisseur.

13. Système selon l'une des revendications 11 à 12, comprenant une jauge de contrainte (13) collée sur ladite portion annulaire (10) présentant la réduction d'épaisseur.

14. Dispositif médical adapté pour être implanté dans un corps humain ou animal, comprenant :
(a) un circuit fluidique comprenant :
- une manchette occlusive gonflable contenant un volume variable d'un fluide, adaptée pour entourer au moins une partie d'un conduit naturel à occlure,
- un réservoir à volume variable rempli d'un fluide, ledit réservoir comprenant une partie fixe et une partie mobile,
- une liaison fluidique entre le réservoir et la manchette occlusive,
(b) un actionneur couplé mécaniquement à la partie mobile du réservoir de sorte à déplacer linéairement ladite partie mobile par rapport à la partie fixe pour ajuster le volume du réservoir,
l'actionneur et le réservoir à volume variable étant agencés dans un boîtier étanche,
(c) un système de protection d'un capteur pour une mesure de force (1) selon l'une des revendications 1 à 13, le capteur de mesure de force (1) étant solidaire de la partie mobile du réservoir à volume variable.

15. Dispositif selon la revendication 14, dans lequel la partie mobile du réservoir à volume variable est un soufflet.

16. Dispositif selon la revendication 15, dans lequel le soufflet comprend une paroi solidaire d'une vis d'entraînement (103), ladite vis d'entraînement étant couplée par une liaison hélicoïdale à une roue dentée (102) apte à être entraînée en rotation par l'actionneur, le capteur de mesure de force étant agencé autour de la roue dentée (102) par l'intermédiaire d'un roulement à billes (104).

17. Dispositif selon la revendication 16, dans lequel la roue dentée (102) et le capteur (1) sont agencés dans une boîte à engrenages (100), le capteur étant maintenu contre l'élément élastique (2) par une bague de fixation (105).

18. Méthode de protection d'un capteur de mesure de force (1) adapté pour mesurer au moins une force de traction ou une force de compression exercée selon un axe longitudinal (X) du capteur et pour être solidarisé à une partie mobile d'un réservoir de fluide, ladite méthode comprenant au moins les étapes consistant à :
- fournir un système de protection dudit capteur selon l'une des revendications 1 à 13,
- exercer une force de traction ou de compression selon l'axe (X) du capteur de sorte que (i) tant que ladite force est inférieure à la force de précontrainte de l'élément élastique, le capteur (1) se déforme pour mesurer la force exercée et (ii) lorsque ladite force devient supérieure à ladite force de précontrainte, le capteur (1) se déplace jusqu'à une butée (106, 107).

19. Méthode selon la revendication 18, dans laquelle le capteur atteint la butée (106, 107) lorsque la force exercée est inférieure à une force de détérioration du capteur.

20. Méthode selon la revendication 18 ou la revendication 19, dans laquelle le capteur ne se déplace vers la butée qu'au-delà de la force de précontrainte.

## Patentansprüche

1. System zum Schutz eines Sensors für eine Kraftmessung, umfassend:
- einen Kraftmesssensor (1), der geeignet ist, mindestens eine Zugkraft oder eine Druckkraft zu messen, die gemäß einer Längsachse (X) des Sensors ausgeübt wird, wobei der Sensor geeignet ist, mit einem beweglichen Teil eines Fluidbehälters fest verbunden zu sein, und
- ein vorgespanntes elastisches Element (2), das eingerichtet ist, um den Kraftsensor (1) in einer Richtung entgegengesetzt zu der ausgeübten Kraft zu belasten, wobei das elastische Element in Richtung der ausgeübten Kraft derart verformbar ist, dass der Sensor vor mindestens einer Druck- oder Zugkraft geschützt wird, die größer als ein Schwellenwert ist.

2. System nach Anspruch 1, das ferner einen Anschlag (106, 107) umfasst, wobei der Kraftmesssensor (1) gemäß der Achse (X) bis zu dem Anschlag in Richtung der Kraft, die gegen die Belastung des elastischen Elements (2) ausgeübt wird, beweglich ist.

3. System nach Anspruch 2, wobei das elastische Element (2) auf einen bestimmten Kraftwert, bezeichnet als Vorspannkraft, vorgespannt ist, wobei die Vorspannkraft kleiner ist als eine maximale Kraft, die von dem Kraftmesssensor aufgenommen werden kann, so dass der Kraftmesssensor geeignet ist, sich erst jenseits der Vorspannkraft in Richtung des Anschlags zu bewegen.

4. System nach einem der Ansprüche 1 bis 3, das ferner ein Zahnrad (102) umfasst, das mit dem Kraftmesssensor (1) fest verbunden ist.

5. System nach einem der Ansprüche 1 bis 4, das ferner ein Kugellager (104) umfasst, das mit dem Kraftmesssensor (1) fest verbunden ist.

6. System nach einem der Ansprüche 2 bis 5, wobei der Anschlag (106) auf einer Seite des Kraftmesssensors (1) eingerichtet ist, die dem Fluidbehälter gegenüberliegt.

7. System nach einem der Ansprüche 1 bis 6, wobei das elastische Element (2) auf einer Seite des Kraftmesssensors (1) eingerichtet ist, die dem Fluidbehälter gegenüberliegt.

8. System nach einem der Ansprüche 1 bis 7, wobei das elastische Element (2) derart mit dem Kraftmesssensor fest verbunden ist, dass eine Messung einer Zug- und/oder Druckkraft durch den Kraftmesssensor ermöglicht wird.

9. System nach einem der Ansprüche 1 bis 8, wobei das vorgespannte elastische Element (2) ein Federring ist.

10. System nach Anspruch 9, wobei der Federring ein gewellter Federring ist.

11. System nach einem der Ansprüche 1 bis 10, wobei der Kraftmesssensor (1) einen ringförmigen Abschnitt (10) mit einer Dickenreduktion umfasst, wobei der Abschnitt (10) unter der Anwendung einer axialen Kraft biegbar ist.

12. System nach Anspruch 11, wobei der Kraftmesssensor (1) mit einem Umfangsabschnitt außerhalb des Abschnitts (10) mit der Dickenreduktion auf dem vorgespannten elastischen Element (2) aufliegt.

13. System nach einem der Ansprüche 11 bis 12, umfassend einen Dehnungsmesser (13), der auf den ringförmigen Abschnitt (10) mit der Dickenreduktion aufgeklebt ist

14. Medizinische Vorrichtung, die zur Implantation in einen menschlichen oder tierischen Körper geeignet ist, umfassend:
(a) einen Fluidkreislauf, umfassend:
- eine aufblasbare Okklusionsmanschette, die ein variables Volumen eines Fluids enthält und geeignet ist, mindestens einen Teil eines zu okkludierenden natürlichen Kanals zu umschließen,
- einen mit einem Fluid gefüllten Behälter mit variablem Volumen, wobei der Behälter einen festen Teil und einen beweglichen Teil umfasst,
- eine Fluidverbindung zwischen dem Behälter und der Okklusionsmanschette,
(b) einen Aktuator, der mit dem beweglichen Teil des Behälters derart mechanisch gekoppelt ist, dass der bewegliche Teil relativ zum festen Teil linear bewegt wird, um das Volumen des Behälters einzustellen,
wobei der Aktuator und der Behälter mit variablem Volumen in einem dichten Gehäuse eingerichtet sind,
(c) ein System zum Schutz eines Kraftmesssensors (1) nach einem der Ansprüche 1 bis 13, wobei der Kraftmesssensor (1) mit dem beweglichen Teil des Behälters mit variablem Volumen fest verbunden ist.

15. Vorrichtung nach Anspruch 14, wobei der bewegliche Teil des Behälters mit variablem Volumen ein Faltenbalg ist.

16. Vorrichtung nach Anspruch 15, wobei der Faltenbalg eine Wand umfasst, die mit einer Antriebsschraube (103) fest verbunden ist, wobei die Antriebsschraube über eine schraubenförmige Verbindung mit einem Zahnrad (102) gekoppelt ist, das durch den Aktuator in Drehung versetzbar ist, wobei der Kraftmesssensor über ein Kugellager (104) um das Zahnrad (102) herum eingerichtet ist.

17. Vorrichtung nach Anspruch 16, wobei das Zahnrad (102) und der Sensor (1) in einem Zahnradgehäuse (100) eingerichtet sind, wobei der Sensor durch einen Befestigungsring (105) gegen das elastische Element (2) gehalten wird.

18. Verfahren zum Schutz eines Kraftmesssensors (1), der geeignet ist, mindestens eine Zugkraft oder eine Druckkraft zu messen, die gemäß einer Längsachse (X) des Sensors ausgeübt wird, und fest mit einem beweglichen Teil eines Fluidbehälters verbunden zu sein, wobei das Verfahren mindestens die folgenden Schritte umfasst:
- Bereitstellen eines Systems zum Schutz des Sensors nach einem der Ansprüche 1 bis 13,
- Ausüben einer Zug- oder Druckkraft gemäß der Achse (X) des Sensors derart, dass (i) so lange die Kraft kleiner ist als die Vorspannkraft des elastischen Elements, sich der Sensor (1) verformt, um die ausgeübte Kraft zu messen, und (ii) wenn die Kraft größer als die Vorspannkraft wird, sich der Sensor (1) bis zu einem Anschlag (106, 107) bewegt.

19. Verfahren nach Anspruch 18, wobei der Sensor den Anschlag (106, 107) erreicht, wenn die ausgeübte Kraft kleiner ist als eine Kraft, die den Sensor beschädigt.

20. Verfahren nach Anspruch 18 oder Anspruch 19, wobei sich der Sensor erst jenseits der Vorspannkraft in Richtung des Anschlags bewegt.

## Claims

1. System for protecting a sensor for a force measurement comprising:
- a force measurement sensor (1), designed to measure at least one tractive force or one compressive force exerted along a longitudinal axis (X) of the sensor, said sensor being designed to be made integral with a moveable part of a fluid reservoir, and
- a pre-strained elastic element (2) arranged to bias the force sensor (1) in a direction opposite to said exerted force, said elastic element being deformable in the direction of the exerted force so as to protect the sensor from at least one compressive or tractive force greater than a threshold.

2. System according to claim 1, further comprising a stop (106, 107), the force measurement sensor (1) being moveable along said axis (X) up to said stop in the direction of the force exerted counter to the biasing of the elastic element (2).

3. System according to claim 2, wherein the elastic element (2) is pre-strained to a determined force value, designated pre-strained force, said pre-strained force being less than a maximum force being able to be withstood by the force measurement sensor, such that the force measurement sensor is designed to be only displaced towards the stop beyond said pre-strained force.

4. System according to one of claims 1 to 3, further comprising a toothed wheel (102) integral with the force measurement sensor (1).

5. System according to one of claims 1 to 4, further comprising a ball bearing (104) integral with the force measurement sensor (1).

6. System according to one of claims 2 to 5, wherein the stop (106) is arranged on a side of the force measurement sensor (1) opposite to the fluid reservoir.

7. System according to one of claims 1 to 6, wherein the elastic element (2) is arranged on a side of the force measurement sensor (1) opposite to the fluid reservoir.

8. System according to one of claims 1 to 7, wherein the elastic element (2) is integral with the force measurement sensor in such a way as to make it possible to measure a tractive and/or compressive force by the force measurement sensor.

9. System according to one of claims 1 to 8, wherein the pre-strained elastic element (2) is a spring washer.

10. System according to claim 9, wherein the spring washer is an elastic wave washer.

11. System according to one of claims 1 to 10, wherein the force measurement sensor (1) comprises an annular portion (10) having a reduction in thickness, said portion (10) being capable of bending under the application of an axial force.

12. System according to claim 11, wherein the force measurement sensor (1) is bearing on the elastic element (2) pre-strained by a peripheral portion external to the portion (10) having the reduction in thickness.

13. System according to one of claims 11 to 12, comprising a strain gauge (13) bonded on said annular portion (10) having the reduction in thickness.

14. Medical device designed to be implanted in a human or animal body, comprising:
(a) a fluidic circuit comprising:
- an inflatable occlusion cuff containing a variable volume of a fluid, designed to surround at least one part of a natural conduit to occlude,
- a variable volume reservoir filled with a fluid, said reservoir comprising a fixed part and a moveable part,
- a fluidic connection between the reservoir and the occlusion cuff,
(b) an actuator mechanically coupled to the moveable part of the reservoir so as to linearly displace said moveable part with respect to the fixed part to adjust the volume of the reservoir,
the actuator and the variable volume reservoir being arranged in a sealed housing,
(c) a system for protecting a sensor for a force measurement (1) according to one of claims 1 to 13, the force measurement sensor (1) being integral with the moveable part of the variable volume reservoir.

15. Device according to claim 14, wherein the moveable part of the variable volume reservoir is a gusset.

16. Device according to claim 15, wherein the gusset comprises a wall integral with a drive screw (103), said drive screw being coupled by a helical connection to a toothed wheel (102) capable of being rotationally driven by the actuator, the force measurement sensor being arranged around the toothed wheel (102) through a ball bearing (104).

17. Device according to claim 16, wherein the toothed wheel (102) and the sensor (1) are arranged in a gear box (100), the sensor being maintained against the elastic element (2) by a fastening ring (105).

18. Method for protecting a force measurement sensor (1) designed to measure at least one tractive force or one compressive force exerted along a longitudinal axis (X) of the sensor and to be made integral with a moveable part of a fluid reservoir, said method comprising at least the steps consisting in:
- providing a system for protecting said sensor according to one of claims 1 to 13,
- exerting a tractive or compressive force along the axis (X) of the sensor such that (i) as long as said force is less than the pre-strained force of the elastic element, the sensor (1) deforms to measure the exerted force and (ii) when said force becomes greater than said pre-strained force, the sensor (1) is displaced up to a stop (106, 107).

19. Method according to claim 18, wherein the sensor reaches the stop (106, 107) when the exerted force is less than a force of deterioration of the sensor.

20. Method according to claim 18 or claim 19, wherein the sensor is only displaced towards the stop beyond the pre-strained force.
